(19) Europäisches Patentamt
European Patent Office
Office européen des brevets

(11) Publication number: **0 144 477 B1**

(12) **EUROPEAN PATENT SPECIFICATION**

(45) Date of publication of patent specification: **18.03.87**

(51) Int. Cl.[4]: **C 07 C 67/08,** C 07 C 69/54, C 07 C 69/24, C 07 C 69/78, C 07 C 69/16, C 07 C 69/618

(21) Application number: **83307576.5**

(22) Date of filing: **13.12.83**

# (54) Method for producing bisphenol derivatives.

(43) Date of publication of application:
**19.06.85 Bulletin 85/25**

(45) Publication of the grant of the patent:
**18.03.87 Bulletin 87/12**

(84) Designated Contracting States:
**CH DE FR GB IT LI NL**

(56) References cited:
**DE-A-2 948 969**
**FR-A-2 264 002**

(73) Proprietor: **SUMITOMO CHEMICAL COMPANY, LIMITED**
**15 Kitahama 5-chome Higashi-ku**
**Osaka-shi Osaka 541 (JP)**

(72) Inventor: **Takahashi, Yokoh**
**2-11-8-208, Sone Higashimachi**
**Toyonaka Osaka-fu (JP)**
Inventor: **Terada, Yutaka**
**8-12, Fukatanicho**
**Nishinomiya Hyogo-ken (JP)**
Inventor: **Yachigo, Shinichi**
**2-11-7-305, Sone Higashimachi**
**Toyonaka Osaka-fu (JP)**
Inventor: **Ishii, Tamaki**
**1-12-2, Uchihonmachi**
**Suita Osaka-fu (JP)**

(74) Representative: **Geering, Keith Edwin et al**
**REDDIE & GROSE 16 Theobalds Road**
**London WC1X 8PL (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to a method for producing bisphenol derivatives represented by the formula (I):

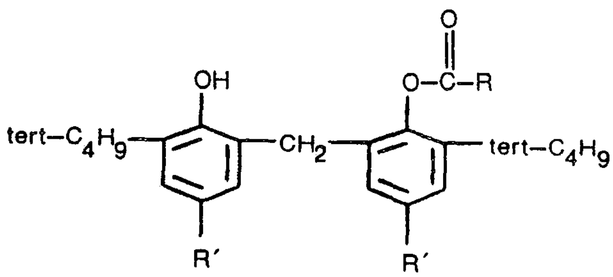

(I)

wherein R represents an alkyl group having 1 to 3 carbon atoms, an alkenyl group having 2 to 4 carbon atoms, or a phenyl or 2-phenylethenyl group, and each R′ is selected from alkyl groups having 1 to 4 carbon atoms.

Bisphenol derivatives represented by the formula (I) are useful as antioxidants. A method for producing them is known in which a carboxylic acid is converted to its acid halide using a halogenating agent such as a chlorinating agent (e.g. thionyl chloride, phosphorus trichloride, phosphorus pentachloride) or brominating agent (e.g. phosphorus tribromide), and the acid halide is then reacted with bisphenol in the presence of a dehydrohalogenating agent (US. Patent No. 3,984,372). Such methods, however, are inevitably carried out in two reaction steps and give a poor yield of the bisphenol derivative based on the carboxylic acid. Furthermore this two-step reaction requires handling of acid halide intermediate which is strongly irritative to eyes, skin, mucous membrane etc.

According to the present invention, there is provided a method for producing bisphenol derivatives represented by the foregoing formula (I) characterized in that carboxylic acid represented by the formula (II):

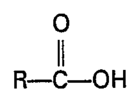

(II)

wherein R is as defined above,
is reacted with bisphenol represented by the formula (III):

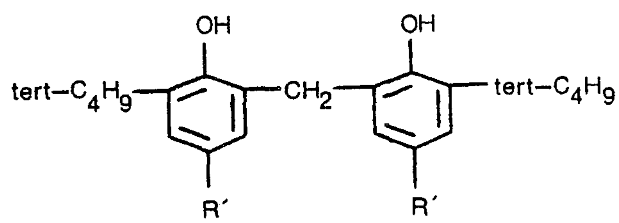

(III)

wherein each R′ is as defined above,
using halogenating agent in the presence of dehydrohalogenating agent.

In the present invention, specific examples of the carboxylic acid represented by the formula (II) include for example acetic acid, propionic acid, butyric acid, acrylic acid, methacrylic acid, crotonic acid, 3-butenoic acid, benzoic acid, cinnamic acid and the like. Specific examples of the bisphenols represented by the formula (III) include for example 2,2′-methylenebis(6-tert-butyl-4-methylphenol), 2,2′-methylenebis(6-tert-butyl-4-ethylphenol), 2,2′-methylenebis(6-tert-butyl-4-propylphenol), 2,2′-methylenebis(6-tert-butyl-4-n-butylphenol), 2,2′-methylenebis(6-tert-butyl-4-sec-butylphenol), and 2,2′-methylenebis(4,6-di-tert-butylphenol).

Specific examples of the dehydrohalogenating agent include for example tertiary amines such as triethylamine, dimethylaniline, N,N-dimethylbenzylamine, tetramethylurea, etc., and pyridine compounds such as pyridine, 4-(N,N-dimethylamino)pyridine, etc. Specific examples of the halogenating agent are for example phosphorus oxychloride, phosphorus oxybromide, o-toluenesulfonyl chloride, p-toluenesulfonyl chloride and the like.

The reaction of the present invention is generally carried out in the presence of a solvent. Suitable solvents include, for example, aliphatic hydrocarbons (e.g. n-hexane, n-heptane), alicyclic ones (e.g. cyclohexane), aromatic ones (e.g. benzene, toluene, xylene), esters (e.g. ethyl acetate, butyl acetate), ethers

(e.g. diethyl ether, tetrahydrofuran, ethylene glycol dimethyl ether), halogenated hydrocarbons (e.g. dichloromethane, chloroform, carbon tetrachloride, 1,2-dichloroethane), and dehydrohalogenating agents which are liquid at room temperature, (e.g. from those above).

In this reaction, the molar ratio of carboxylic acid to bisphenol is preferably 0.7—1.5:1, more preferably 0.9—1.2:1.

The amount of halogenating agent used is suitably 0.5 to 1.0 mole, preferably 0.6 to 0.9 mole, more preferably 0.65 to 0.8 mole, per mole of the carboxylic acid for phosphorus oxychloride and phosphorus oxybromide, and is suitably 0.8 to 1.6 moles, preferably 0.9 to 1.2 moles, per mole of the acid for o- or p-toluenesulfonyl chloride.

The optimum amount of dehydrohalogenating agent used depends upon the kind of halogenating agent used. When the halogenating agent is phosphorus oxychloride or phosphorus oxybromide, said amount is suitably 2.7 to 3.6 equivalents, preferably 2.9 to 3.2 equivalents, per mole of the halogenating agent. When the halogenating agent is o- or p-toluenesulfonyl chloride, said amount is suitably 0.8 to 2.4 equivalents, preferably 0.9 to 2.2 equivalents, per mole of the halogenating agent. The expression "equivalent" is used because some dehydrohalogenating agents, for example, tetramethylurea, will catch two hydrogen halide molecules per one molecule of agent.

The reaction temperature is suitably −20°C to 150°C, preferably 0°C to 120°C, more preferably 0°C to 100°C.

After completion of the reaction, the objective product can be separated from the reaction solution by various methods; for example, optionally after separating the acid salt of the dehydrohalogenating agent by filtration, the reaction solution is neutralized as need arises and washed with water, and the solvent is removed by evaporation from the organic layer; or water is added to the reaction solution which is then cooled, and the deposited crystal is separated. The objective product obtained may further be purified by well-known methods such as recrystallization, washing with solvents, and the like.

By the present invention, bisphenol derivatives represented by the formula (I) can be produced commercially in one step and in good yields from carboxylic acids without handling a strongly irritative acid halide.

The present invention is illustrated by the following Examples:

### Example 1

To a 2-liter four-necked flask equipped with a thermometer, stirring apparatus, condenser and dropping funnel were added 340.51 g (1.0 mole) of 2,2′-methylenebis(6-tert-butyl-4-methylphenol), 72.06 g (1.0 mole) of acrylic acid, 500 g of toluene and 209.47 g (2.07 moles) of triethylamine. After replacing the air in the container with nitrogen, 105.8 g (0.69 mole) of phosphorus oxychloride was added dropwise with stirring. After completion of the dropwise addition, the reaction solution was kept at 80°C for 1 hour. Thereafter, the organic layer was washed with water until it became neutral, and toluene was removed under reduced pressure. The residue was then recrystallized from n-hexane to obtain 374.8 g of a white crystalline 2,2′-methylenebis(6-tert-butyl-4-methylphenol) monoacrylate having a melting point of 132° to 134°C. Yield, 95%.

### Example 2

To the same flask as used in Example 1 were added 340.51 g (1.0 mole) of 2,2′-methylenebis(6-tert-butyl-4-methylphenol), 72.06 g (1.0 mole) of acrylic acid, 400 g of n-heptane and 212.5 g (2.10 moles) of triethylamine. After replacing the air in the container with nitrogen, 200.7 g (0.70 mole) of phosphorus oxybromide was added dropwise with stirring. After completion of the dropwise addition, the reaction solution was kept at 80°C for 1 hour. Thereafter, 500 g of water was added, and after cooling to room temperature, the deposited product was filtered off. The product obtained was washed with water until the washings became neutral to obtain 379.6 g of 2,2′-methylenebis(6-tert-butyl-4-methylphenol) monoacrylate as white crystals. Melting point, 132°—134°C. Yield, 96%.

### Example 3

To the same flask as used in Example 1 were added 340.51 g (1.0 mole) of 2,2′-methylenebis(6-tert-butyl-4-methylphenol), 86.41 g (1.0 mole) of 98.5% methacrylic acid, 500 g of toluene and 242.86 g (2.4 moles) of triethylamine. After replacing the air in the container with nitrogen, 229.14 g (1.2 moles) of o-toluenesulfonyl chloride was added dropwise with stirring. After completion of the dropwise addition, the reaction solution was kept at 90°C for 1 hour. Thereafter, the organic layer was washed with water until it became neutral, and toluene was removed under reduced pressure. The residue was recrystallized from n-hexane to obtain 384.1 g of a white crystalline 2,2′-methylenebis(6-tert-butyl-4-methylphenol) monomethacrylate having a melting point of 144° to 146°C. Yield 94%.

### Example 4

To the same flask as used in Example 1 were added 340.51 g (1.0 mole) of 2,2′-methylenebis(6-tert-butyl-4-methylphenol), 74.08 g (1.0 mole) of propionic acid, 500 g of toluene and 191.42 g (2.42 moles) of pyridine. After replacing the air in the container with nitrogen, 210.05 g (1.1 moles) of o-toluenesulfonyl chloride was added dropwise with stirring. After completion of the dropwise addition, the reaction solution

was kept at 100°C for 1 hour and then after-treated in the same manner as in Example 2 to obtain 384.7 g of a white crystalline 2,2'-methylenebis(6-tert-butyl-4-methylphenol) monopropionate having a melting point of 108° to 110°C. Yield, 97%.

Example 5

To the same flask as used in Example 1 were added 340.51 g (1.0 mole) of 2,2'-methylenebis(6-tert-butyl-4-methylphenol), 122.12 g (1.0 mole) of benzoic acid, 500 g of toluene and 242.86 g (2.4 moles) of triethylamine. After replacing the air in the container with nitrogen, 229.15 g (1.2 moles) of o-toluenesulfonyl chloride was added dropwise with stirring. After completion of the dropwise addition, temperature maintenance and after-treatment were carried out in the same manner as in Example 3 to obtain 426.83 g of a white crystalline 2,2'-methylenebis(6-tert-butyl-4-methylphenol) monobenzoate having a melting point of 149° to 151°C. Yield, 96%.

Example 6

To a 2-liter four-necked flask equipped with a thermometer, stirring apparatus, condenser and feed inlet were added 340.51 g (1.0 mole) of 2,2'-methylenebis(6-tert-butyl-4-methylphenol), 60.05 g (1.0 mole) of acetic acid and 800 g of triethylamine. After replacing the air in the container with nitrogen, 305.52 g (1.6 moles) of p-toluenesulfonyl chloride was added little by little with stirring. After completion of the addition, the reaction solution was kept at 60°C for 1 hour with stirring, and then triethylamine hydrochloride formed was filtered off at room temperature. The triethylamine hydrochloride was further washed with toluene, and the filtrate and toluene washing were mixed and washed with water until the mixture became neutral. Thereafter, the solvent was removed under reduced pressure, and the residue was recrystallized from n-hexane to obtain 344.3 g of a white crystalline 2,2'-methylenebis(6-tert-butyl-4-methylphenol) monoacetate having a melting point of 98° to 104°C. Yield, 90%.

Example 7

To the same flask as used in Example 6 were added 340.51 g (1.0 mole) of 2,2'-methylenebis(6-tert-butyl-4-methylphenol), 151.18 g (1.0 mole) of cinnamic acid, 500 g of toluene and 118.65 g (1.5 moles) of pyridine. After replacing the air in the container with nitrogen, 286.43 g (1.5 moles) of p-toluenesulfonyl chloride was added little by little with stirring. After completion of the addition, temperature maintenance and after-treatment were carried out in the same manner as in Example 6, and the residue was recrystallized from toluene to obtain 404.8 g of a white crystalline 2,2'-methylenebis(6-tert-butyl-4-methylphenol) monocinnamoate having a melting point of 164° to 169°C. Yield, 86%.

Comparative example 1

To a 500-ml four-necked flask equipped with a thermometer, stirring apparatus, condenser and dropping funnel were added 144.1 g (2.0 moles) of 99.8% acrylic acid, 2.0 g (0.03 mole) of N,N-dimethylformamide and 1.44 g (0.01 mole) of hydroquinone, and 238.0 g (2.0 moles) of thionyl chloride was added dropwise with stirring while maintaining the inner temperature at 50°C. After completion of the dropwise addition, temperature maintenance was carried out for 30 minutes, 0.14 g (0.0013 mole) of cuprous chloride was then added and the product was distilled under normal pressure to collect a fraction between 71°C and 85°C. Thus, 70.60 g of acryloyl chloride was obtained. Yield, 39%.

To a 500-ml four-necked flask equipped with a thermometer, stirring apparatus, condenser and dropping funnel were added 82.0 g (0.241 mole) of 2,2'-methylenebis(6-tert-butyl-4-methylphenol), 200 g of toluene and 23.9 g (0.290 mole) of triethylamine. After replacing the air in the container with nitrogen, 25 g (0.276 mole) of acryloyl chloride was added dropwise with stirring. After completion of the dropwise addition, stirring was continued for 1 hour, excess triethylamine was neutralized with dilute hydrochloric acid and the formed triethylamine hydrochloride was then removed by washing with water. Toluene was then removed by evaporation from the toluene layer after completion of the washing, and the residue obtained was recrystallized from 20 g of n-hexane to obtain 90.0 g of 2,2'-methylenebis(6-tert-butyl-4-methylphenol) monoacrylate as white crystals. Melting point, 132°—134°C. Yield, 95%.

The yield of the product based on acrylic acid was 37.1%.

Comparative example 2

To a 2-liter four-necked flask equipped with a thermometer, stirring apparatus, condenser and dropping funnel were added 340.51 g (1.00 mole) of 2,2'-methylenebis(6-tert-butyl-4-methylphenol), 500 g of toluene and 113.41 g (1.12 moles) of triethylamine. After replacing the air in the container with nitrogen, 93.28 g (1.01 mole) of acryloyl chloride was added dropwise with stirring.

After completion of the dropwise addition, stirring was continued for 1 hour, excess triethylamine was converted to its hydrochloride with dilute hydrochloric acid, and the formed triethylamine hydrochloride was removed by washing with water. After washing with water, toluene was removed by evaporation from the toluene layer, and the residue obtained was recrystallized from 425 g of n-hexane to obtain 286.96 g of 2,2'-methylenebis(6-tert-butyl-4-methylphenol) monoacrylate as white crystals. Melting point, 132°—134°C. Yield, 72.7%.

The yield of the product based on acrylic acid was 28.3%.

Comparative example 3

Propionyl chloride was obtained from propionic acid and benzoyl chloride according to the method described in J.A.C.S., *60*, 1325 (1938). Yield, 89%. Boiling point, 77°—78.5°C.

To a 300-ml four-necked flask equipped with a thermometer, stirring apparatus and condenser were added 34.51 g (0.10 mole) of 2,2'-methylenebis(6-tert-butyl-4-methylphenol), 50 g of toluene and 11.13 g (0.11 mole) of triethylamine. After replacing the air in the container with nitrogen, 10.20 g (0.11 mole) of propionyl chloride was added dropwise. After completion of the dropwise addition, after-treatment was carried out in the same manner as in Comparative example 1 to obtain 35.0 g of 2,2'-methylenebis(6-tert-butyl-4-methylphenol) monopropionate as white crystals. Melting point, 108°—111°C. Yield, 88.4%.

The yield of the product based on propionic acid was 78%.

**Claims**

1. A method for producing a bisphenol derivative represented by the formula (I),

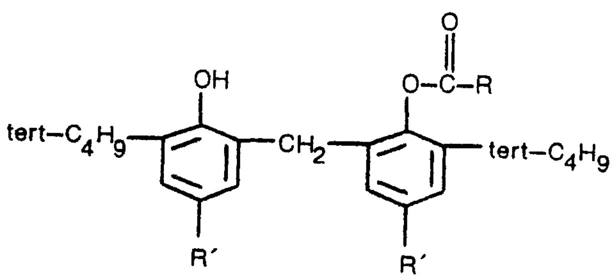

(I)

wherein R represents an alkyl group having 1 to 3 carbon atoms, an alkenyl group having 2 to 4 carbon atoms, or a phenyl or 2-phenylethenyl group, and each R' is selected from alkyl groups of 1 to 4 carbon atoms, characterised in that carboxylic acid represented by the formula (II),

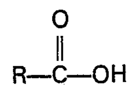

(II)

wherein R is as defined above, is reacted with bisphenol represented by the formula (III),

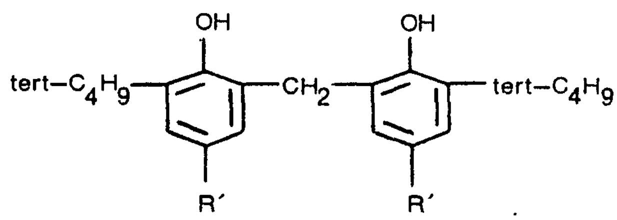

(III)

wherein each R' is as defined above, using halogenating agent in the presence of dehydrohalogenating agent.

2. A method according to claim 1 wherein the carboxylic acid is selected from acetic acid, propionic acid, butyric acid, acrylic acid, methacrylic acid, crotonic acid, 3-butenoic acid, benzoic acid and cinnamic acid.

3. A method according to claim 1 or 2 wherein the bisphenol is selected from 2,2'-methylenebis(6-tert-butyl-4-methylphenol), 2,2'-methylenebis(6-tert-butyl-4-ethylphenol), 2,2'-methylenebis(6-tert-butyl-4-propylphenol), 2,2'-methylenebis(6-tert-butyl-4-n-butylphenol), 2,2'-methylenebis(6-tert-butyl-4-sec-butylphenol) and 2,2'-methylenebis(4,6-di-tert-butylphenol).

4. A method according to any preceding claim wherein the dehydrohalogenating agent is a tertiary amine (e.g. selected from triethylamine, dimethylaniline, N,N-dimethylbenzylamine and tetramethylurea) or pyridine compound (e.g. selected from pyridine and 4-(N,N-dimethylamino)pyridine).

5. A method according to any preceding claim wherein the molar ratio of carboxylic acid to bisphenol is 0.7—1.5:1.

6. A method according to any preceding claim wherein the halogenating agent is phosphorus oxychloride or phosphorus oxybromide, preferably in an amount of 0.5 to 1.0 mole per mole of carboxylic acid.

7. A method according to any of claims 1 to 5 wherein the halogenating agent is o- or p-toluenesulfonyl chloride, preferably in an amount of 0.8 to 1.6 moles per mole of carboxylic acid.

8. A method according to claim 6 wherein the amount of dehydrohalogenating agent used is 2.7 to 3.6 equivalents per mole of halogenating agent.

9. A method according to claim 7 wherein the amount of dehydrohalogenating agent used is 0.8 to 2.4 equivalents per mole of halogenating agent.

10. A method according to any preceding claim wherein the reaction temperature is from −20°C to 150°C.

**Patentansprüche**

1. Verfahren zur Herstellung eines Bisphenolderivates der Formel (I)

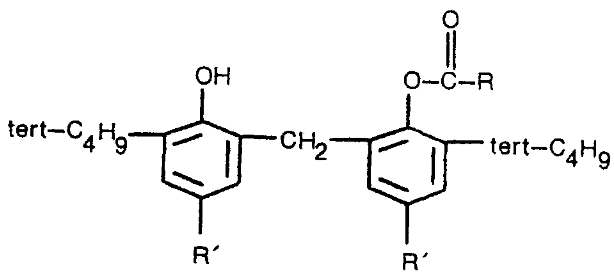

(I)

worin R eine Alkylgruppe mit 1 bis 3 Kohlenstoffatomen, eine Alkenylgruppe mit 2 bis 4 Kohlenstoffatomen oder eine Phenyl- und 2-Phenylethenylgruppe ist und jeweils R′ ausgewählt ist aus Alkylgruppen mit 1 bis 4 Kohlenstoffatomen, dadurch gekennzeichnet, daß eine Carbonsäure der Formel (II)

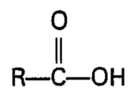

(II)

worin R wie vorstehend definiert ist, mit einem Bisphenol der Formel (III)

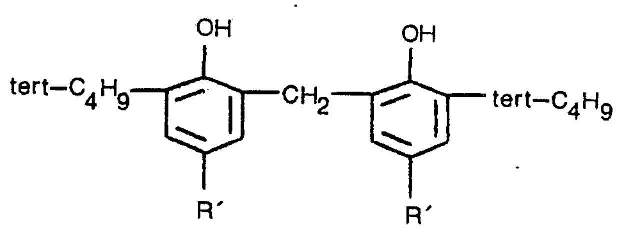

(III)

worin jeweils R′ die vorstehend angegebene Definition besitzt, unter Verwendung eines Halogenierungsmittels in Gegenwart eines Dehydrohalogenierungsmittels umgesetzt wird.

2. Verfahren nach Anspruch 1, dadurch gekennzeichnet, daß die Carbonsäure ausgewählt wird aus Essigsäure, Propionsäure, Buttersäure, Acrylsäure, Methacrylsäure, Crotonsäure, 3-Butensäure, Benzoesäure und Zimtsäure.

3. Verfahren nach Anspruch 1 oder 2, dadurch gekennzeichnet, daß das Bisphenol ausgewählt wird aus 2,2′-Methylen-bis-(6-tert.-butyl-4-methylphenol), 2,2′-Methylen-bis-(6-tert.-butyl-4-ethylphenol), 2,2′-Methylen-bis-(6-tert.-butyl-4-propylphenol), 2,2′-Methylen-bis-(6-tert.-butyl-4-n-butylphenol), 2,2′-Methylen-bis-(6-tert.-butyl-4-sec.-butylphenol) und 2,2′-Methylen-bis-(4,6-di-tert.-butylphenol).

4. Verfahren nach einem der vorhergehenden Ansprüche, dadurch gekennzeichnet, daß das Dehydrohalogenierungsmittel ein tertiäres Amin (beispielsweise ausgewählt aus Triethylamin, Dimethylanilin, N,N-Dimethylbenzylamin und Tetramethylharnstoff) oder eine Pyridinverbindung (beispielsweise ausgewählt aus Pyridin und 4-(N,N-dimethylamino)pyridin) ist.

5. Verfahren nach einem der vorhergehenden Ansprüche, dadurch gekennzeichnet, daß das Molverhältnis der Carbonsäure zu dem Bisphenol 0,7 bis 1,5:1 beträgt.

6. Verfahren nach einem der vorhergehenden Ansprüche, dadurch gekennzeichnet, daß das Halogenierungsmittel Phosphoroxychlorid oder Phosphoroxybromid, vorzugsweise in einer Menge von 0,5 bis 1,0 Mol pro Mol der Carbonsäure, ist.

7. Verfahren nach einem der Ansprüche 1 bis 5, dadurch gekennzeichnet, daß das Halogenierungsmittel aus o- oder p-Toluolsulfonylchlorid, vorzugsweise in einer Menge von 0,8 bis 1,6 Mol pro Mol der Carbonsäure, besteht.

8. Verfahren nach Anspruch 6, dadurch gekennzeichnet, daß die Menge des verwendeten Dehydrohalogenierungsmittels 2,7 bis 3,6 Äquivalente pro Mol des Halogenierungsmittels beträgt.

9. Verfahren nach Anspruch 7, dadurch gekennzeichnet, daß die Menge des verwendeten Dehydrohalogenierungsmittels 0,8 bis 2,4 Äquivalente pro Mol des Halogenierungsmittels beträgt.

10. Verfahren nach einem der vorhergehenden Ansprüche, dadurch gekennzeichnet, daß die Reaktionstemperatur −20°C bis 150°C beträgt.

**Revendications**

1. Procédé de production d'un dérivé de bisphénol représenté par la formule (I),

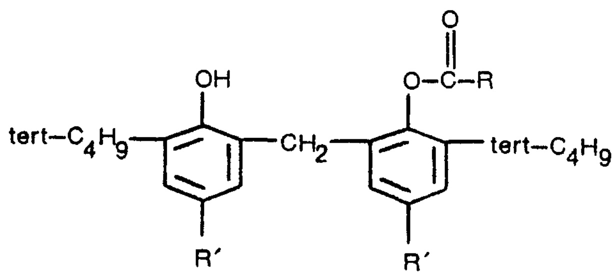

(I)

dans laquelle R représente un groupe alkyle ayant 1 à 3 atomes de carbone, un groupe alcényle ayant 2 à 4 atomes de carbone, un groupe phényle ou un groupe 2-phényléthényle, et chaque R′ est choisi entre des groupes alkyle ayant 1 à 4 atomes de carbone, caractérisé en ce que l'acide carboxylique représenté par la formule (II)

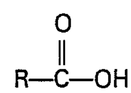

(II)

dans laquelle R est tel que défini ci-dessus, est amené à réagir avec un bisphénol représenté par la formule (III)

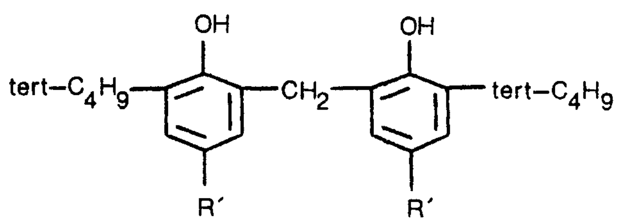

(III)

dans laquelle R′ est tel que défini ci-dessus, en utilisant un agent d'halogénation en présence d'un agent de déshydrohalogénation.

2. Procédé suivant la revendication 1, dans lequel l'acide carboxylique est choisi entre l'acide acétique, l'acide propionique, l'acide butyrique, l'acide acrylique, l'acide méthacrylique, l'acide crotonique, l'acide 3-buténoïque, l'acide benzoïque et l'acide cinnamique.

3. Procédé suivant la revendication 1 ou 2, dans lequel le bisphénol est choisi entre le 2,2′-méthylènebis(6-tertiobutyl-4-méthylphénol), le 2,2′-méthylènebis(6-tertiobutyl-4-éthylphénol), le 2,2′-méthylènebis(6-tertiobutyl-4-propylphénol), le 2,2′-méthylènebis(6-tertiobutyl-4-n-butylphénol), le 2,2′-méthylènebis(6-tertiobutyl-4-sec-butylphénol) et le 2,2′-méthylènebis(4,6-di-tertiobutylphénol).

4. Procédé suivant l'une quelconque des revendications précédentes, dans lequel l'agent de déshydrohalogénation est une amine tertiaire (choisie, par exemple, entre la triéthylamine, la diméthylaniline, la N,N-diméthylbenzylamine et la tétraméthylurée) ou un composé pyridique (choisi par exemple entre la pyridine et la 4-(N,N-diméthylamino)pyridine).

5. Procédé suivant l'une quelconque des revendications précédentes, dans lequel le rapport molaire de l'acide carboxylique au bisphénol est égal à 0,7—1,5:1.

6. Procédé suivant l'une quelconque des revendications précédentes, dans lequel l'agent d'halogénation est l'oxychlorure de phosphore ou l'oxybromure de phosphore, de préférence en une quantité de 0,5 à 1,0 mole par mole d'acide carboxylique.

7. Procédé suivant l'une quelconque des revendications 1 à 5, dans lequel l'agent d'halogénation est le chlorure d'ortho- ou de paratoluènesulfonyle, de préférence en une quantité de 0,8 à 1,6 mole par mole d'acide carboxylique.

8. Procédé suivant la revendication 6, dans lequel la quantité d'agent de déshydrohalogénation utilisée va de 2,7 à 3,6 équivalents par mole d'agent d'halogénation.

9. Procédé suivant la revendication 7, dans lequel la quantité d'agent de déshydrohalogénation utilisée va de 0,8 à 2,4 équivalents par mole d'agent d'halogénation.

10. Procédé suivant l'une quelconque des revendications précédentes, dans lequel la température réactionnelle va de −20°C à 150°C.